# EUROPEAN PATENT APPLICATION

(11) **EP 4 659 762 A1**
(43) Date of publication of application: **10.12.2025**
(21) Application number: 24750318.8
(22) Date of filing: 31.01.2024
(51) Int. Cl.: A61K 45/00, A61K 31/711, A61K 39/395, A61K 48/00, A61P 1/16, A61P 29/00, A61P 43/00, C07K 16/24, C12N 15/113

(54) **PHARMACEUTICAL COMPOSITION FOR PREVENTING AND TREATING HEPATIC FIBROGENESIS**

(30) Priority: 03.02.2023 JP 2023015045
(71) Applicant: Educational Foundation Of Osaka Medical And Pharmaceutical University, Takatsuki-shi, Osaka 569-8686 (JP)
(72) Inventor: ASAI Akira, Takatsuki City, Osaka 569-8686 (JP)
(74) Representative: Hautier IP - MC/EP
(86) International application number: PCT/JP2024/002979
(87) International publication number: WO 2024/162369

(57) **Abstract**

A pharmaceutical composition for preventing and treating hepatic fibrosis is provided, the pharmaceutical composition targeting various cells (e.g., macrophages, Kupffer cells, T cells), having reduced side effects, and being effective. A pharmaceutical composition according to the present disclosure is a pharmaceutical composition for preventing and treating hepatic fibrosis and includes a CCL1 inhibitor.

## Description

### TECHNICAL FIELD

The present invention relates to pharmaceutical compositions for preventing and treating hepatic fibrosis and specifically relates to a pharmaceutical composition configured to inhibit the activity of CCL1 which is a chemokine.

### BACKGROUND ART

Liver cirrhosis causes various complications and is thus known to have a poor prognosis. What substantially underlies the liver cirrhosis is hepatic fibrosis from a variety of causes such as virus, alcohol, and non-alcoholic steatohepatitis (NASH). Individual treatments for these various causes are available, and there are means for indirectly preventing the hepatic fibrosis. However, there have been no pharmacological agents relating to the prevention and/or treatment of the hepatic fibrosis itself which is common to the various causes. The hepatic fibrosis is comprehensively caused when activation of hepatic stellate cells and/or myofibroblastic cells due to chronic inflammation in the liver brings about excessive production of an extracellular matrix such as collagen and inhibition of matrix metalloprotease (a collagen-fiber degrading enzyme). Research on the hepatic stellate cells and myofibroblastic cells which cause the fibrosis has been, and is, conducted, but no effective treatments have been found. This is because various approaches that may inhibit the hepatic fibrosis are not directed to specific cells and/or substances which cause the fibrosis of the liver, and therefore, those treatments affect on the whole body, thereby attenuating their effects and causing various side effects.

Macrophages, a type of cell present in microtissue included in the liver, are called Kupffer cells. These cells are immune cells that play a central role in the chronic inflammation in the liver, and these cells are known to produce various cytokines in accordance with the chronic inflammation in the liver, activate the hepatic stellate cells and/or the myofibroblastic cells, and play an important role in the process of the hepatic fibrosis. The macrophages include various types of disease-specifically activated macrophages. In general, the activated macrophages are broadly classified into M1 macrophages and M2 macrophages. The M1 macrophages secrete cytokines such as Interleukin (IL)-12 and Interferon (INF)-γ, thereby triggering inflammation. In contrast, the M2 macrophages produce cytokines such as Transforming Growth Factor (TGF)-β and IL-10 and activate the myofibroblastic cells, thereby serving for a tissue repair. The M2 macrophages are further sub-classified into M2a macrophages, M2b macrophages, and M2c macrophages. It is known that the M2a macrophages and the M2c macrophages link their fate to the disappearance/growth of the IL-4, whereas the M2b macrophages are independent of environmental changes and remain active for a long period of time while producing, by themselves, CCL1 known as a chemokine (Non-Patent Literature 1). It has recently revealed that the Kupffer cells in some liver damaged, or liver cirrhosis, hosts have the properties of the M2b macrophage (Non-Patent Literatures 2 and 3).

### CITATION LIST

### NON-PATENT LITERATURE

[Non-Patent Literature 1] Journal of Leukocyte Biology., Vol 92(2012), p859-867.
[Non-Patent Literature 2] Journal of Leukocyte Biology., Vol 109(2021), p943-952.
[Non-Patent Literature 3] Oncoimmunology., Vol 6(2017), e1299301.

### SUMMARY OF INVENTION

### TECHNICAL PROBLEM

As explained above, the relationship of the Kupffer cells to the hepatic fibrosis is known, but the relationship has not yet advantageously used to create effective medical drugs for preventing and treating the hepatic fibrosis.

In view of the foregoing, it is an object of the present disclosure to provide a pharmaceutical composition for preventing and treating the hepatic fibrosis, the pharmaceutical composition targeting various cells such as the Kupffer cells, other macrophages, and T cells, having reduced side effects, and being effective.

### SOLUTION TO PROBLEM

To achieve the object, the present inventor conducted intensive studies and consequently found that most of the Kupffer cells present in liver tissue of liver cirrhosis patients are M2b macrophages (IL-10 + CCL1 + cells) that produce IL-10 and C-C motif chemokine Ligand 1 (CCL1). The inventors found that inhibiting this CCL1 (inhibiting the production of CCL1 or inhibiting the activity of CCL1 thus produced) can prevent the hepatic fibrosis and can also reduce hepatic fibers, and thereby, the inventors has accomplished the present invention.

Specifically, a pharmaceutical composition according to the present disclosure is a pharmaceutical composition for preventing and treating hepatic fibrosis, the pharmaceutical composition including a CCL1 inhibiting substance (e.g., an antibody to CCL1, antisense oligonucleotide, nucleic acid including RNA).

### ADVANTAGEOUS EFFECTS OF INVENTION

The pharmaceutical composition according to the present invention includes the substance which inhibits CCL1, and therefore, the pharmaceutical composition is capable of inhibiting CCL1 production from various cells (also including the Kupffer cells) or the activity of CCL1 thus produced and consequently preventing and treating the hepatic fibrosis.

### BRIEF DESCRIPTION OF DRAWINGS

[FIG. 1] FIG. 1 is a photograph showing a result of Hematoxylin-Eosin (HE) staining of a slice of liver tissue obtained from chronic liver damage mice of a carbon tetrachloride-treated group in an example.
[FIG. 2] FIG. 2 is a photograph showing a result of sirius red staining of a slice of liver tissue obtained from chronic liver damage mice of the carbon tetrachloride-treated group in the example.
[FIG. 3] FIG. 3 is a photograph showing a result of Hematoxylin-Eosin (HE) staining of a slice of liver tissue obtained from chronic liver damage mice of a CCL1 inhibitor-treated group in the example.
[FIG. 4] FIG. 4 is a photograph showing a result of sirius red staining of a slice of liver tissue obtained from chronic liver damage mice of the CCL1 inhibitor-treated group in the example.
[FIG. 5] FIG. 5 is a graph showing a result of evaluation of the inflammatory activity (Activity) and the stage of fibrosis (Fibrosis) based on Modified HAI grading and also evaluation of the incidence of hepatic fibrosis, from the results of the staining of the tissue slices of FIGS. 1 to 4.
[FIG. 6] FIG. 6 is a graph showing a result of comparison of the incidence of hepatic fibrosis among two types of CCL1 inhibitor-treated groups (anti-CCL1 antibody or CCL1 antisense oligonucleotide (AS ODN) using liver cirrhosis mice in the example and a phosphate buffered saline (PBS)-treated group for a control purpose.

### DESCRIPTION OF EMBODIMENTS

An embodiment of the present invention will be described below. The description of a preferable embodiment below is substantially a mere example and does not intend to limit the present invention, the application method thereof, or the application thereof.

An embodiment of the present invention is a pharmaceutical composition for preventing and treating hepatic fibrosis, the pharmaceutical composition including a substance intended to inhibit CCL1.

As explained above, the hepatic fibrosis is comprehensively caused when activation of hepatic stellate cells and/or myofibroblastic cells due to chronic inflammation occurring in the liver brings about excessive production of an extracellular matrix such as collagen and inhibition of matrix metalloprotease which is an enzyme that degrades collagen fibers. Moreover, Kupper cells are known as immune cells relating to the chronic inflammation in the liver, and the Kupper cells produce various cytokines in accordance with the chronic inflammation in the liver, activate the hepatic stellate cells and/or the myofibroblastic cells, and play an important role in the process of the hepatic fibrosis. Most of the Kupffer cells present in liver tissue of liver cirrhosis patients are M2b macrophages (IL-10 + CCL1 + cells) that produce IL-10 and CCL1.

The macrophages include various types of disease-specifically activated macrophages. In general, the activated macrophages are broadly classified into M1 macrophages and M2 macrophages. The M1 macrophages secrete cytokines such as IL-12 and INF-γ, thereby triggering inflammation. In contrast, the M2 macrophages produce cytokines such as TGF-β and IL-10 and activate the myofibroblastic cells, thereby serving for a tissue repair. The M2 macrophages are further sub-classified into M2a macrophages, the M2b macrophages, and M2c macrophages. It is known that the M2a macrophages and the M2c macrophages link their fate to the disappearance/growth of the IL-4, whereas the M2b macrophages are independent of environmental changes and remain active for a long period of time while producing, by themself, an essential cytokine (CCL1). Since the Kupffer cells, which have the properties of the M2b macrophages, are dominantly found in the liver tissue of the liver cirrhosis patients, these cells presumably control the hepatic fibrosis, and therefore, the present inventor considered that a drug development targeting only the M2b macrophages has a high degree of specificity and would thus lead to drug development applications with less side effects.

Most activated Kupffer cells present in the chronically inflamed liver are Kupffer cells derived from peripheral blood-derived monocytes reactively infiltrated in accordance with inflammations, and these Kupffer cells have the properties of the M2b macrophages. The M2a macrophages and the M2c macrophages, which are other types of activated M2 macrophages, may temporarily be activated but act to reduce the inflammations and eventually decrease in the activity, and the properties thereof disappear in a short period of time. However, the M2b macrophages, which are most of the Kupffer cells in the liver cirrhosis host, remain present for a long period of time and continue activating the hepatic stellate cells and/or the myofibroblastic cells, thereby strongly promoting the hepatic fibrosis.

There have been a large number of hepatic fibrosis studies directed to the macrophage, but none of the studies has resulted in the drug development. One of the reasons for this is that none of these studies is directed to activated Kupffer cells present specifically in the liver cirrhosis host. Candidate substances used in these studies are not directed to only the Kupffer cells which are to cause the hepatic fibrosis, and therefore, the candidate substances also inhibit various types of macrophages present in the whole body other than the liver, which consequently attenuates the effect thereof and causes various side effects in the whole body. Based on these backgrounds, the present inventor focused on, and targets, a disease-specific property of the Kupffer cells present in liver cirrhosis, and thereby, the present inventor conceived of the development of a novel treatment that inhibits only the Kupffer cells which are target cells.

Further, the present inventor, at this time, concentrates on CCL1 which is a type of chemokine. CCL1 is secreted from activated M2b macrophages and binds to a cell having Chemokine Receptor 8, thereby activating macrophages, Th2 cells, regulatory T cells, and the like. The present inventor revealed, in a preceding study, that modifying CCL1 productivity of the M2b macrophages with a gene therapy drastically reduces the M2b macrophages and induces the appearance of quiescent macrophages (see Non-Patent Literature 1). Specifically, a CCL1 inhibitor modifies the CCL1 productivity of the M2b macrophages and reverses the activated M2b macrophages to the quiescent macrophages to also suppress their activation together with a large number of locally distributed macrophages. Moreover, the T cells are directly inhibited, thereby suppressing the hepatic fibrosis. Based on the knowledge, the present invention uses the CCL1 inhibitor to regulate the Kupffer cells, the T cells, and the like having the properties of the M2b macrophages found specifically to hosts in which the hepatic fibrosis is observed, thereby inhibiting the activity of CCL1 to eventually suppress the hepatic fibrosis.

CCL1 antisense oligonucleotide (AS ODN) which is one of the CCL1 inhibitors used in the present embodiment is not limited to a particular agent as long as it can suppress the production of CCL1 in the Kupffer cells. For example, in the case of mice, CCL1 AS ODN including SEQ ID NO: 1 indicated below can be used, and in the case of humans, CCL1 AS ODN including SEQ ID NOs: 2 to 23 indicated below can be used.
SEQ ID NO: 1: GAAGCCCGAGAACATCAT
SEQ ID NO: 2: CAGCTAGCAGCAAGCACA
SEQ ID NO: 3: GATGATCTGCATGTCTTC
SEQ ID NO: 4: TGTGGTGATGATCTGCA
SEQ ID NO: 5: GGGCTGTGGTGATGATCT
SEQ ID NO: 6: AAGCACACCAGGGCTGT
SEQ ID NO: 7: CATCTGGAGAAGGGTACCTG
SEQ ID NO: 8: CATTGGAGCAGATGGAGCTG
SEQ ID NO: 9: AAGCCATGTGGTTTCCAGAG
SEQ ID NO: 10: AAGGAATGGTGTAGGGCTGG
SEQ ID NO: 11: CAGAGGGTTGGGGGTTGATG
SEQ ID NO: 12: GCCATGTGGTTTCCAGAG
SEQ ID NO: 13: GAGAAGGGTACCTGCATG
SEQ ID NO: 14: CAACATCTGGAGAAGGGT
SEQ ID NO: 15: GTCTTCTGGTCTGGCTTG
SEQ ID NO: 16: ATGTCTTCTGGTCTGGCT
SEQ ID NO: 17: CATGTCTTCTGGTCTGGC
SEQ ID NO: 18: TGTGGTGATGATCTGCAT
SEQ ID NO: 19: CTGTGGTGATGATCTGCA
SEQ ID NO: 20: AGGGCTGTGGTGATGATC
SEQ ID NO: 21: GGGCTGTGGTGATGATC
SEQ ID NO: 22: AGGGCTGTGGTGATGAT
SEQ ID NO: 23: CAGGGCTGTGGTGATGAT

In the present embodiment, the CCL1 inhibitor may be an agent that includes an anti-CCL1 antibody or an RNA nucleic acid preparation other than CCL1 AS ODN. The anti-CCL1 antibody or the RNA nucleic acid preparation is not limited to a particular antibody or preparation as long as it can inhibit the production, or the activity, of CCL1.

The pharmaceutical composition according to the present embodiment includes the CCL1 inhibitor as a main component and may include various additives. Examples of the additives include sterilized water, physiologic saline, vegetable oils, emulsifiers, suspending agents, surfactants, stabilizer, flavoring agent, excipients, pH stabilizers, vehicles, preservatives, and binding agents. The type of the additives is not limited to a particular type.

In the present embodiment, the formulation of the pharmaceutical composition is not limited to a particular formulation. The pharmaceutical composition may be in the form of an oral agent or may be in the form of an injection product. Examples of the oral agent include tablets, encapsulated formulations, granules, and sublingual formulations.

In the present embodiment, the administration method of the pharmaceutical composition is not limited to a particular method. For example, the pharmaceutical composition may be orally administered or injected.

In the present embodiment, examples of the target to which the pharmaceutical composition is to be applied include, but are not limited to, mammals, such as humans, mice, cows, goat, pigs, dogs, and cats.

### EXAMPLES

An example will be shown below to describe the pharmaceutical composition according to the present invention in detail. In the present example, the effect of the CCL1 inhibitor on chronic liver damage mice was considered. Methods of experiments and results thereof will be described below.

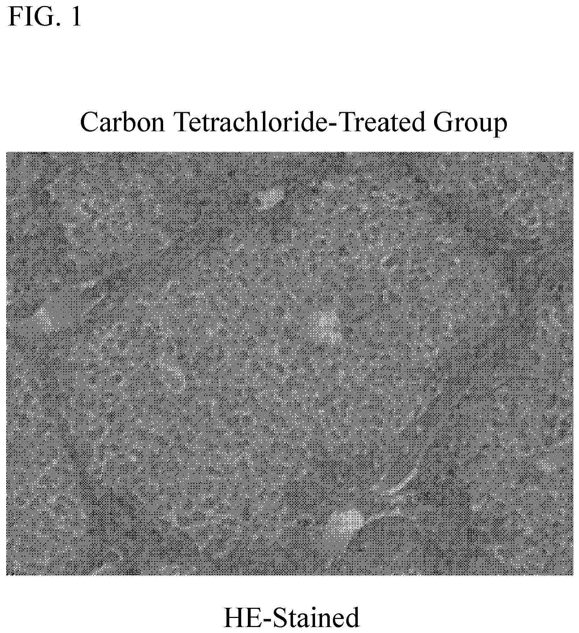

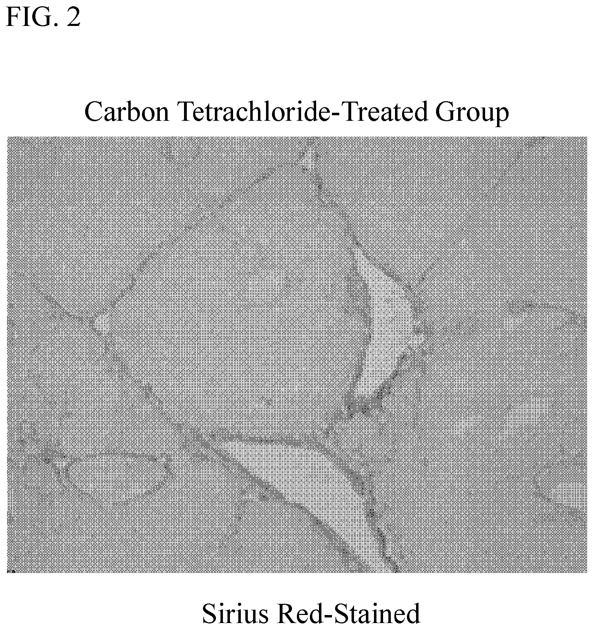

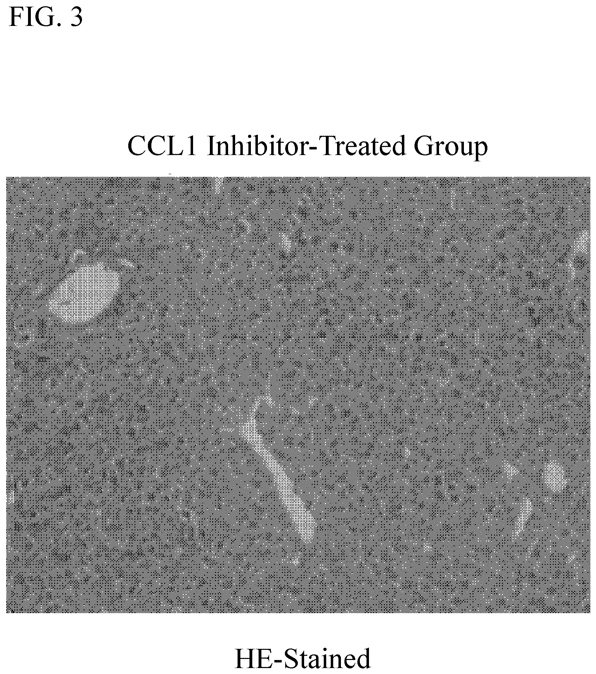

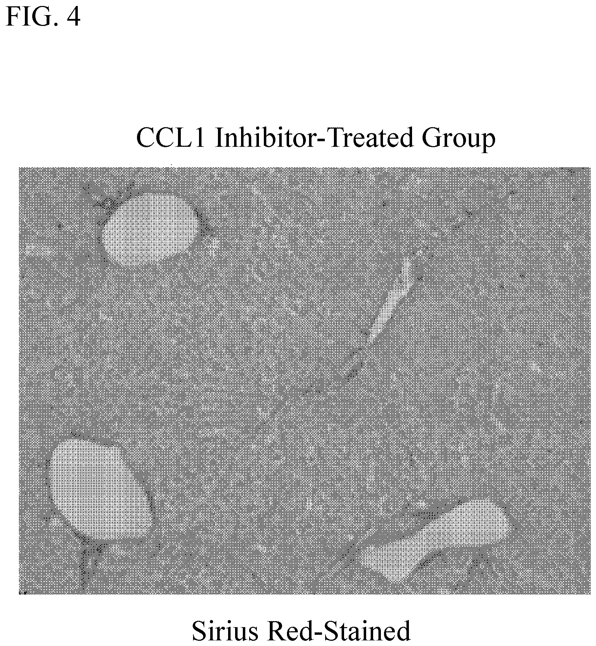

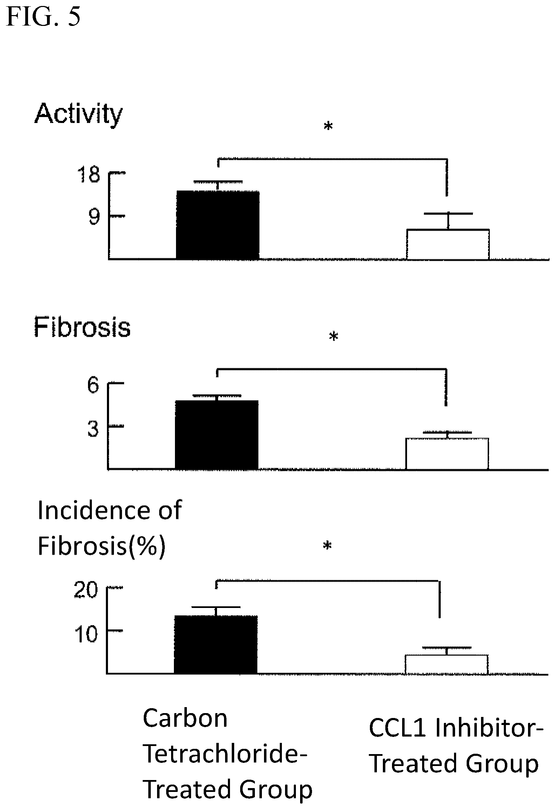

To C57/BL6 mice, subcutaneous administration of carbon tetrachloride (1.0 mg/kg/twice per week) for 8 weeks produces chronic liver damage mice in which hepatic fibrosis is observed, and subcutaneous administration of the carbon tetrachloride for 12 weeks makes liver cirrhosis mice. In the present example, mice (carbon tetrachloride-treated group) produced by subcutaneously administering, to the chronic liver damage mice obtained by the subcutaneous administering of the carbon tetrachloride for 8 weeks as described above, the carbon tetrachloride (1.0 mg/kg/twice per week) for additional 4 weeks (12 weeks in total) were compared with mice (CCL1 inhibitor-treated group) produced by subcutaneously administering, concurrently with the administration of the carbon tetrachloride to the chronic liver damage mice for the additional 4 weeks, a CCL1 inhibitor (CCL1 AS ODN of SEQ ID NO: 1, 6.0 µg/kg/twice per week) to another site of the chronic liver damage mice. Specifically, after 12 weeks of the administration had elapsed, liver tissue was taken out of each mouse by a conventional method to prepare liver tissue slices of the liver tissue, the liver tissue slices were each HE-stained or sirius red-stained (FIGS. 1 to 4), the inflammatory activity (Activity) and the stage of fibrosis (Fibrosis) of each of the liver tissue slices were evaluated based on well-known Modified HAI grading, and The incidence of the hepatic fibrosis in each of the liver tissue slices was further evaluated by using BZ-X710 (microscope) and HALO (image analysis software) (FIG. 5).

As shown in FIGS. 1 to 5, almost no inflammation or hepatic fibrosis was observed and the effect of preventing the inflammation and the hepatic fibrosis was confirmed in the mice administered with the CCL1 inhibitor as compared with the mice administered with only the carbon tetrachloride.

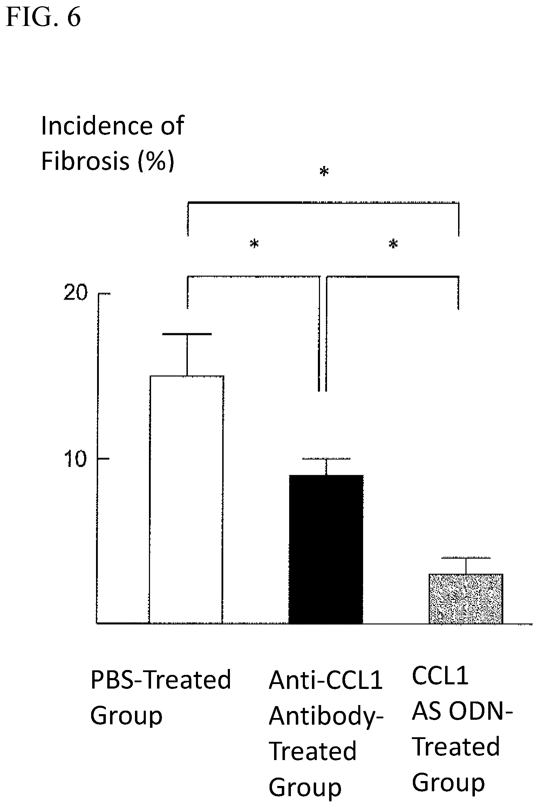

Then, the effect of the CCL1 inhibitor on the liver cirrhosis mice produced by subcutaneously administering the carbon tetrachloride (1.0 mg/kg/twice per week) to the C57/BL6 mice for 12 weeks was evaluated. Specifically, the fibrosis was evaluated in comparison among the anti-CCL1 antibody-treated group of the liver cirrhosis mice (Mouse CCL1/I-309/TCA-3 Antibody, R&D SYSTEMS,#MAB8451, 10 µg/mouse/three times per week, subcutaneous injection, 4 weeks), the anti-CCL1 AS ODN-treated group of the liver cirrhosis mice (CCL1 AS ODN of SEQ ID NO: 1, 6.0 µg/kg/twice per week, subcutaneous injection, 4 weeks), and the PBS-treated group of the liver cirrhosis mice (a control group of the above groups). The results are shown in FIG. 6.

As shown in FIG. 6, inhibiting the activity of CCL1 by various methods significantly suppressed the hepatic fibrosis in each of the groups as compared with the group administered with PBS. In particular, the suppression of the hepatic fibrosis in the CCL1 AS ODN-treated group was significant as compared with the anti-CCL1 antibody-treated group.

From the results above, it can be said that inhibiting CCL1 reduces the inflammation in the liver and suppress the hepatic fibrosis. Further, the inhibiting of CCL1 can provide the effect of reducing the fibrosis also in the cirrhotic liver.

## Claims

1. A pharmaceutical composition for preventing and treating hepatic fibrosis, the pharmaceutical composition comprising a CCL1 inhibitor.

2. The pharmaceutical composition of claim 1, wherein the CCL1 inhibitor includes an anti-CCL1 antibody or CCL1 AS ODN including any one of SEQ ID NOs: 1 to 23.
